# EUROPEAN PATENT APPLICATION

(11) **EP 4 591 865 A1**
(43) Date of publication of application: **30.07.2025**
(21) Application number: 23868621.6
(22) Date of filing: 21.09.2023
(51) Int. Cl.: A61K 31/513, A61K 31/198, A61K 31/223, A61K 31/20, A61K 45/06, A61P 35/00, A61K 9/00, A61K 39/395, A61K 9/06

(54) **PHARMACEUTICAL COMPOSITION FOR DISRUPTING TUMOR BLOOD VESSELS**

(30) Priority: 22.09.2022 KR 20220120038; 30.11.2022 KR 20220163840; 20.09.2023 KR 20230125780
(71) Applicant: Samsung Medical Foundation, Seoul 03181 (KR)
(72) Inventor: PARK, Jung Ho, Seoul 03165 (KR)
(74) Representative: Wichmann, Hendrik
(86) International application number: PCT/KR2023/014416
(87) International publication number: WO 2024/063569

(57) **Abstract**

An exemplary embodiment of the pharmaceutical composition for tumor vascular disruption may include alkaline 5-fluorouracil and a nitric oxide synthase inhibitor. For example, the inclusion of alkaline 5-fluorouracil and a nitric oxide synthase inhibitor may induce apoptosis of tumor vascular endothelial cells and promote tumor vascular occlusion, thereby providing superior anticancer efficacy.

## Description

### BACKGROUND

### 1. Field

The present invention relates to a pharmaceutical composition for tumor vascular disruption, which may be used in the medical and pharmaceutical fields.

### 2. Description of the Related Art

Fluorouracil (5-FU) is a chemotherapeutic agent that is widely used for the treatment of various cancers. 5-Fluorouracil is an analogue of uracil, which is naturally present in the human body, and exerts its anticancer effects primarily by inhibiting DNA synthesis and repair through the suppression of thymidylate synthase, as well as by interfering with RNA function.

5-Fluorouracil is primarily administered intravenously, requiring direct administration in a hospital setting, and is associated with adverse effects such as thrombosis. To address these issues, oral prodrug formulations of 5-FU have been developed in recent years.

Similar to conventional anticancer agents, 5-fluorouracil exerts its anticancer effects by inhibiting intracellular nucleic acid synthesis or directly binding to nucleic acids, thereby impairing their function. However, 5-fluorouracil is not selectively cytotoxic to cancer cells but also damages normal cells, particularly actively dividing tissue cells, leading to frequent myelosuppression and gastrointestinal toxicity due to phosphorylation in the gastrointestinal tract. Additionally, although the incidence of cardiotoxicity is relatively low, severe and potentially life-threatening cases have been reported in 1.5% to 18% of patients.

Accordingly, research on anticancer agents that maintain the therapeutic efficacy of 5-fluorouracil while reducing adverse effects is necessary. For example, Korean Patent Publication No. 10-2013-0074325 discloses a pharmaceutical composition for cancer treatment comprising 5-fluorouracil; however, this composition does not simultaneously provide both reduced adverse effects and enhanced anticancer efficacy.

### SUMMARY

The objective of the present invention is to provide a pharmaceutical composition for tumor vascular disruption that exhibits superior anticancer efficacy while reducing adverse effects.

An exemplary embodiment of the pharmaceutical composition for tumor vascular disruption may include alkaline 5-fluorouracil and a nitric oxide synthase inhibitor.

In one embodiment, the nitric oxide synthase inhibitor may include one or more selected from L-NMMA (NG-Monomethyl-L-arginine), L-NAME (NG-Nitro-L-arginine methyl ester), L-NA (Nitro Arginine), and 7NI (Nitroindazole).

In one embodiment, the nitric oxide synthase inhibitor may include L-NMMA (NG-Monomethyl-L-arginine).

In one embodiment, the alkalinity may be in the range of pH 8 to 9.

In one embodiment, the alkaline 5-fluorouracil (5-FU) may be in a form in which 5-fluorouracil is dissolved in an alkaline solvent.

In one embodiment, the concentration of the alkaline 5-fluorouracil may be in the range of 0.1 to 600 mM in the total composition.

In one embodiment, the alkaline 5-fluorouracil may increase thrombospondin-1 protein expression in tumor vascular endothelial cells, thereby inducing apoptosis of the endothelial cells.

In one embodiment, the concentration of the nitric oxide synthase inhibitor may be in the range of 0.1 to 300 mM in the total composition.

In one embodiment, the nitric oxide synthase inhibitor may constrict tumor blood vessels and promote thrombosis within the tumor vasculature damaged by 5-fluorouracil, thereby occluding the blood vessels.

In one embodiment, the composition may further include one or more selected from bevacizumab, capric acid or a pharmaceutically acceptable salt thereof, and poloxamer.

In one embodiment, the composition may further include one or more selected from cytotoxic anticancer agents, targeted anticancer agents, and immuno-oncology agents.

In one embodiment, the tumor may include a solid tumor.

In one embodiment, the composition may be administered by local injection around the tumor.

In one embodiment, the area around the tumor may be a submucosal layer where tumor blood vessels are located.

An exemplary embodiment of a local formulation for solid tumor administration may include the pharmaceutical composition for tumor vascular disruption.

The pharmaceutical composition for tumor vascular disruption according to the present invention may include alkaline 5-fluorouracil and a nitric oxide synthase inhibitor.

For example, when alkaline 5-fluorouracil is injected into the base of a tumor, it may effectively induce apoptosis of vascular endothelial cells, thereby disrupting the tumor. This effect results from the destruction of blood vessels supplying the tumor rather than directly targeting the tumor itself, and thus may be applicable to other cancer types.

The pharmaceutical composition for tumor vascular disruption according to the present invention, comprising alkaline 5-fluorouracil and a nitric oxide synthase inhibitor, may induce apoptosis of tumor vascular endothelial cells and promote tumor vascular occlusion, thereby exhibiting superior anticancer efficacy.

Furthermore, the pharmaceutical composition for tumor vascular disruption according to the present invention may also reduce adverse effects associated with anticancer agents.

### BRIEF DESCRIPTION OF THE DRAWINGS

FIG.1 illustrates an example of the local injection of a pharmaceutical composition for tumor vascular destruction around the tumor according to an embodiment.
FIG.2 presents the results of a Western blot experiment confirming the expression of TSP-1 protein in the HUVEC cell line.
FIG.3 shows the results of TSP-1 expression in HUVEC cells according to the concentration and exposure time of 5-FU.
FIG.4 presents the results of measuring LDH release in HUVEC cells according to the concentration and exposure time of 5-FU.
FIG.5 shows the effect of cell death in HUVEC cells according to the pH of 5-FU.
FIG.6 illustrates the results of CD31 staining of gastric cancer masses one week after the administration of alkaline 5-FU (right) compared to the control group (left).
FIG.7 illustrates the results of VEGF staining of gastric cancer masses one week after the administration of alkaline 5-FU (right) compared to the control group (left).
FIG.8 presents a photograph showing the tumor size after the administration of alkaline 5-FU following the xenotransplantation of human gastric cancer cells into nude mice.
FIG.9 presents a graph showing the tumor size (volume) after the administration of alkaline 5-FU following the xenotransplantation of human gastric cancer cells into nude mice.
FIG.10a and FIG.10b illustrate the pathological changes observed in the surrounding tissues when alkaline 5-FU was injected around the tumor following the xenotransplantation of human gastric cancer cells into nude mice.
FIG.11 presents the results of confirming the effect of cell death when L-NMMA and alkaline 5-FU are mixed.
FIG.12 presents a photograph showing the tumor size (volume) after the administration of L-NMMA and alkaline 5-FU following the xenotransplantation of human gastric cancer cells into nude mice.
FIG.13a and FIG.13b present graphs showing the tumor size (volume) after the administration of L-NMMA and alkaline 5-FU following the xenotransplantation of human gastric cancer cells into nude mice.
FIG.14 presents a photograph showing the tumor size (volume) after the administration of bevacizumab following the xenotransplantation of human gastric cancer cells into nude mice.
FIG.15 presents a photograph showing the tumor size (volume) after the administration of bevacizumab and alkaline 5-FU following the xenotransplantation of human gastric cancer cells into nude mice.
FIG.16 presents a graph showing the tumor size (volume) after the administration of bevacizumab and alkaline 5-FU following the xenotransplantation of human gastric cancer cells into nude mice.
FIG.17 illustrates a graph representing the viscosity of P407 according to temperature and viscosity.
FIG.18 presents a graph showing the results of a drug release test of 5-FU-sol-gel.
FIG.19 presents a photograph showing the tumor size (volume) after the administration of 30% P407 and alkaline 5-FU following the xenotransplantation of human gastric cancer cells into nude mice.
FIG.20 presents a graph showing the tumor size (volume) after the administration of 30% P407 and alkaline 5-FU following the xenotransplantation of human gastric cancer cells into nude mice.

### DETAILED DESCRIPTION OF THE EMBODIMENTS

An exemplary embodiment of the pharmaceutical composition for tumor vascular disruption (hereinafter referred to as the 'composition') includes alkaline 5-fluorouracil (5-FU) and a nitric oxide synthase inhibitor. Accordingly, the composition may enhance apoptosis of vascular endothelial cells near cancer cells and promote vascular occlusion, thereby providing superior anticancer efficacy

Hereinafter, the composition according to exemplary embodiments of the present invention will be described in detail with reference to the drawings and embodiments. However, the drawings and embodiments are merely exemplary and should not be construed as limiting the scope of the present invention.

An exemplary embodiment of the composition may include alkaline 5-fluorouracil (5-FU).

In one embodiment, the alkaline 5-fluorouracil (5-FU) may increase the expression of thrombospondin-1 (TSP-1) protein in tumor vascular endothelial cells, thereby inducing apoptosis of the endothelial cells.

When fluorouracil is mixed with an alkaline solvent, synergy may be exhibited. For example, when mixed with an alkaline solvent, a superior anticancer effect may be provided compared to when mixed with a neutral or acidic solvent. For example, alkaline 5-fluorouracil may target vascular endothelial cells in tumor tissue and induce cell death. For example, when alkaline 5-fluorouracil is injected into the lower portion of tumor tissue, apoptosis of vascular endothelial cells supplying blood to the tumor may be induced, leading to the destruction of blood vessels and necrosis of the tumor. In this case, the pH of 5-fluorouracil is an important factor in inducing the apoptosis of vascular endothelial cells, and when the pH is weakly alkaline, for example, in the range of 8.4 to 9.0, apoptosis of vascular endothelial cells may be significantly increased compared to neutral or acidic 5-fluorouracil.

In other words, alkaline 5-fluorouracil destroys blood vessels near cancer cells and inhibits angiogenesis, blocking and suppressing the supply of nutrients or oxygen to cancer cells, thereby exhibiting an anticancer effect. The effect results from the destruction of blood vessels supplying blood to the tumor rather than targeting the tumor itself, allowing application to other cancer types. Additionally, adverse effects of anticancer drugs can be reduced.

In one embodiment, the alkaline 5-fluorouracil may be in a form in which 5-fluorouracil is dissolved in an alkaline solvent. The alkaline solvent may include, for example, alkaline water or physiological saline.

In one embodiment, the alkaline pH may range from 8.0 to 9.0. For example, the pH range may be 8.0 to 9.0, 8.2 to 9.0, 8.4 to 9.0, or 8.4 to 8.8. However, the pH is not limited to these ranges.

In some embodiments, the concentration of alkaline 5-fluorouracil may be selected without restriction by a person skilled in the art. For example, the concentration in the total composition may range from 0.1 mM to 600 mM, 0.1 mM to 500 mM, 0.5 mM to 390 mM, 1 mM to 350 mM, 10 mM to 300 mM, 50 mM to 250 mM, or 100 mM to 200 mM. However, the concentration is not limited to these ranges.

An exemplary embodiment of the composition may include alkaline 5-fluorouracil and a nitric oxide synthase inhibitor.

The nitric oxide synthase inhibitor may include, for example, L-NMMA (N^{G}-Monomethyl-L-arginine), L-NAME (NG-Nitro-L-arginine methyl ester), L-NA (Nitro Arginine), or 7NI (Nitroindazole).

Nitric oxide (NO) is synthesized and secreted by NO synthase in vascular endothelial cells, leading to vasodilation. Additionally, NO protects cells by preventing vascular endothelial cells from undergoing apoptosis in response to external stimuli such as lipopolysaccharide (LPS), angiotensin II, caspase-3 overexpression, and TNF-alpha.

In some embodiments, the nitric oxide synthase inhibitor may constrict tumor blood vessels and promote thrombosis within the tumor vasculature damaged by 5-FU. For example, alkaline 5-fluorouracil may damage or destroy tumor vascular endothelial cells, leading to thrombus formation inside the tumor vasculature. The nitric oxide synthase inhibitor may facilitate the occlusion of such tumor blood vessels, thereby more effectively inducing tumor necrosis.

In some embodiments, the nitric oxide synthase inhibitor is preferably L-NMMA (N^{G}-Monomethyl-L-arginine).

L-NMMA is a non-specific NO synthase inhibitor that effectively increases blood pressure when administered into blood vessels. Under physiological conditions, inhibition of NO synthase by L-NMMA alone does not induce platelet activation in vivo. However, when endothelial cell damage occurs in tumor vasculature, L-NMMA may promote platelet activation and thrombosis. In other words, damaged vascular endothelial cells may be induced to form thrombi. Accordingly, the combination of L-NMMA and 5-fluorouracil may induce both vascular endothelial cell apoptosis and tumor vascular occlusion, thereby exhibiting superior anticancer efficacy.

For example, the concentration of the nitric oxide synthase inhibitor may range from 0.1 mM to 300 mM, 0.1 mM to 250 mM, 1 mM to 200 mM, or 10 mM to 200 mM in the total composition. However, the present invention is not limited thereto.

As another example, L-NMMA may be included in an amount of 0.5 mg/kg to 20 mg/kg, 1 mg/kg to 10 mg/kg, 2 mg/kg to 8 mg/kg, or 4 mg/kg to 6 mg/kg based on the weight of the subject receiving treatment. However, the present invention is not limited thereto.

In one embodiment, the composition may further include at least one selected from bevacizumab, capric acid or a pharmaceutically acceptable salt thereof, and poloxamer.

Bevacizumab is a recombinant humanized monoclonal antibody designed to inhibit vascular endothelial growth factor (VEGF), which promotes angiogenesis.

Bevacizumab is conventionally used as an anticancer agent either alone or in combination with other drugs. Traditionally, bevacizumab has been primarily administered via intravenous injection. In the case of advanced gastric cancer, reports indicate that combining fluoropyrimidine-cisplatin therapy with bevacizumab improves progression-free survival and overall response rate.

However, in one embodiment, the composition may further include bevacizumab in a sustained-release form. By inhibiting tumor angiogenesis, the formulation may effectively eliminate blood vessels distributed within the tumor.

Capric acid or a pharmaceutically acceptable salt thereof, and poloxamer exhibit low toxicity, ensuring biocompatibility and superior in vivo stability. For example, combining capric acid or a pharmaceutically acceptable salt thereof with poloxamer may increase viscosity and elevate the sol-gel transition temperature. For instance, the sol-gel transition temperature may range between room temperature and body temperature, for example, from 20 to 40°C or from 25 to 37°C. Within this temperature range, the formulation remains in a sol state at room temperature, facilitating storage, transport, and use. Upon administration, the transition into a gel state enhances diffusion within tissues.

In some embodiments, the weight ratio of capric acid to poloxamer may range from 0.25 to 3.4:30, 1 to 3.4:30, or 2 to 3:30. Specifically, the weight ratio of capric acid to poloxamer may range from 2.75 to 3.3:30 or from 2.8 to 3.2:30. Within these ranges, the sol-gel transition of the carrier is likely to occur between 25°C and 36°C.

The capric acid is a compound represented by the following Chemical Formula 1 and is a type of saturated fatty acid.

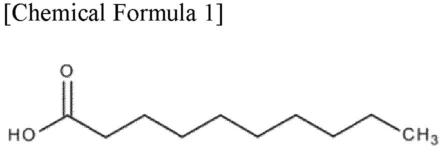

The pharmaceutically acceptable salt may be a salt prepared using capric acid and a relatively non-toxic acid or base. The pharmaceutically acceptable salt may include, for example, a metal salt or an acid addition salt.

The metal salt may be a sodium, potassium, or calcium salt. The metal salt may be prepared using a base. For example, an alkali metal or alkaline earth metal salt may be obtained by dissolving the compound in an excess of an alkali metal hydroxide or alkaline earth metal hydroxide solution, filtering the resulting insoluble compound salt, and evaporating and/or drying the filtrate.

An acid addition salt may be prepared from an inorganic acid such as hydrochloric acid, nitric acid, phosphoric acid, sulfuric acid, hydrobromic acid, hydroiodic acid, nitrous acid, or phosphorous acid, and from a salt of a non-toxic organic acid such as an aliphatic mono- and dicarboxylate, a phenyl-substituted alkanoate, a hydroxy alkanoate, an alkandioate, an aromatic acid, or an aliphatic or aromatic sulfonic acid. These physiologically non-toxic salts may include sulfates, pyrosulfates, bisulfates, sulfites, bisulfites, nitrates, phosphates, monohydrogen phosphates, dihydrogen phosphates, metaphosphates, pyrophosphates, chlorides, bromides, iodides, fluorides, acetates, propionates, decanoates, caprylates, acrylates, formates, isobutyrates, caprates, heptanoates, propylenates, oxalates, malonates, succinates, suberates, sebacates, fumarates, maleates, butene-1,4-dioates, hexane-1,6-dioates, benzoates, chlorobenzoates, methylbenzoates, dinitrobenzoates, hydroxybenzoates, methoxybenzoates, phthalates, terephthalates, benzene sulfonates, toluene sulfonates, chlorobenzene sulfonates, xylene sulfonates, phenylacetates, phenylpropionates, phenylbutyrates, citrates, lactates, β-hydroxybutyrates, glycolates, maleates, tartrates, methanesulfonates, propanesulfonates, naphthalene-1-sulfonates, naphthalene-2-sulfonates, or mandelates.

The poloxamer may be a triblock copolymer comprising a poly(ethylene oxide)-poly(propylene oxide)-poly(ethylene oxide) (PEO-PPO-PEO) structure. For example, the poloxamer may be represented by the following Chemical Formula 2.

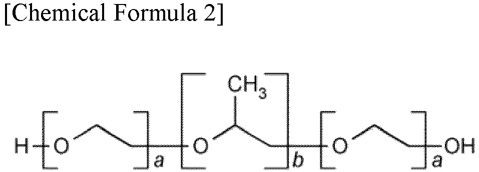

The molecular weight of the poloxamer may be, for example, a weight-average molecular weight in the range of 1,000 to 100,000, 10,000 to 100,000, 10,000 to 20,000, or 10,000 to 15,000. The molecular weight may be appropriately selected by a person skilled in the art depending on the substance to be delivered.

Poloxamers are generally represented using a numbering system that indicates the approximate molecular weight and the percentage content of polyoxyethylene and are also referred to by the trade name Pluronic.

For example, the poloxamer may be Poloxamer 101, Poloxamer 105, Poloxamer 108, Poloxamer 122, Poloxamer 123, Poloxamer 124, Poloxamer 181, Poloxamer 182, Poloxamer 183, Poloxamer 184, Poloxamer 185, Poloxamer 188, Poloxamer 212, Poloxamer 215, Poloxamer 217, Poloxamer 231, Poloxamer 234, Poloxamer 235, Poloxamer 237, Poloxamer 238, Poloxamer 282, Poloxamer 284, Poloxamer 288, Poloxamer 331, Poloxamer 333, Poloxamer 334, Poloxamer 335, Poloxamer 338, Poloxamer 401, Poloxamer 402, Poloxamer 403, and Poloxamer 407, and the like.

The poloxamer is a type of surfactant and may form micelles in an aqueous environment, allowing the loading of substances. A hydrogel composed of poloxamers may form a matrix and may be used as a carrier for the localized and sustained release of substances. The poloxamer hydrogel may include cross-linking between poloxamer molecules. The poloxamer may be used in various molecular weights and ratios.

In one embodiment, the composition may further include a known anticancer agent. For example, the composition may include at least one selected from a cytotoxic anticancer agent, a targeted anticancer agent, and an immuno-oncology agent.

A cytotoxic anticancer agent may interfere with the metabolic pathways of cancer cells to inhibit DNA or RNA synthesis and division or may induce cell death by directly damaging DNA. The cytotoxic anticancer agent may refer to a chemotherapeutic agent or a chemical drug-based anticancer agent. For example, the cytotoxic anticancer agent may be an alkylating agent, a platinum-based compound, an antimetabolite, or a plant-derived alkaloid. However, the present invention is not limited thereto, as long as the anticancer effect is provided by a chemical agent.

For example, an alkylating agent refers to a substance capable of introducing an alkyl group (R-CH₂) into another compound. Examples of alkylating agents include cyclophosphamide, ifosfamide, and bendamustine.

For example, a platinum-based compound contains platinum and refers to a substance that forms oxides, chlorides, or coordination complexes. Examples of platinum-based compounds include cisplatin, carboplatin, and oxaliplatin.

For example, an antimetabolite refers to a substance that inhibits the growth and proliferation of cells by antagonizing essential metabolites necessary for the metabolism or growth of tumor cells. Examples of antimetabolites include methotrexate, cladribine, fludarabine, pemetrexed, and mercaptopurine.

For example, a plant-derived alkaloid refers to a compound containing basic nitrogen that exhibits strong physiological activity in animals and is derived from plant extracts. Examples of plant-derived alkaloids include docetaxel, cabazitaxel, paclitaxel, vincristine, and vinblastine."

A targeted anticancer agent may exert anticancer effects by interfering with molecular activities involved in cancer growth and development, targeting proteins or genes that are specifically altered in cancer cells or cancer tissues. For example, the targeted anticancer agent may be a tyrosine kinase inhibitor, a PARP (poly-ADP ribose polymerase) inhibitor, a CDK4/6 (cyclin-dependent kinase 4/6) inhibitor, or an antibody-drug conjugate.

Unlike conventional anticancer agents that directly attack cancer cells, an immuno-oncology agent may function as a therapeutic agent that stimulates the immune system by introducing artificial immune proteins into the body, thereby enabling immune cells to selectively attack only cancer cells. For example, the immuno-oncology agent may include immune checkpoint inhibitors, immune cell therapies, or therapeutic antibodies for passive immunotherapy, and cancer treatment vaccines or immunomodulators for active immunotherapy. However, the present invention is not limited thereto.

The additional component may further include other carriers and may be formulated together with a carrier. A person skilled in the art may appropriately select the additional component in consideration of the type of bioactive substance and the administration route of the carrier.

In some embodiments, the composition may be a sustained-release formulation.

The longer the exposure time to vascular endothelial cells, the greater the apoptotic effect on vascular endothelial cells. To provide the composition as a sustained-release formulation, the composition may further include capric acid or a pharmaceutically acceptable salt thereof, and poloxamer.

In one embodiment, bevacizumab may be added to the composition and provided in a sustained-release form. By inhibiting angiogenesis in cancer cells, blood vessels distributed within the tumor can be effectively eliminated.

A composition according to some embodiments may include one or more active ingredients that exhibit the same or similar functions in relation to tumor treatment.

A composition according to some embodiments may further include a compound that maintains or enhances the solubility and/or absorbability of the active ingredient.

In one embodiment, the tumor may include a solid tumor.

In some embodiments, the tumor may include, for example, gastric cancer, liver cancer, pancreatic cancer, osteosarcoma, skin cancer, lung cancer, neuroblastoma, uterine cancer, kidney cancer, prostate cancer, breast cancer, colorectal cancer, bile duct cancer, bladder cancer, ovarian cancer, brain tumor, cervical cancer, prostate cancer, testicular cancer, penile cancer, genitourinary cancer, seminoma, esophageal cancer, laryngeal cancer, gastrointestinal cancer, keratoacanthoma, follicular carcinoma, melanoma, small cell lung carcinoma, non-small cell lung carcinoma (NSCLC), pulmonary adenocarcinoma, squamous cell carcinoma of the lung, colon cancer, thyroid cancer, papillary carcinoma, cholangiocarcinoma, renal cancer, bone cancer, bone marrow disorders, hairy cell leukemia, oral and pharyngeal cancer, lip cancer, tongue cancer, oral cancer, salivary gland cancer, pharyngeal cancer, small intestine cancer, colon cancer, rectal cancer, vulvar cancer, thyroid cancer, endometrial cancer, central nervous system cancer, peritoneal cancer, hepatocellular carcinoma, head cancer or neck cancer. However, the present invention is not limited thereto.

The formulation of the composition may be prepared as an oral or parenteral formulation. For example, the formulation may be suitable for oral, rectal, nasal, topical (including buccal and sublingual), subcutaneous, vaginal, or parenteral (including intramuscular, subcutaneous, and intravenous) administration. Alternatively, the formulation may be suitable for administration by inhalation or insufflation.

The formulation of the composition may be an injectable formulation. The formulation does not form precipitation in biological environments, such as blood, and allows administration through a fine injection needle.

The formulation of the composition is preferably an injectable formulation.

In one embodiment, the composition may be administered locally around the tumor via injection.

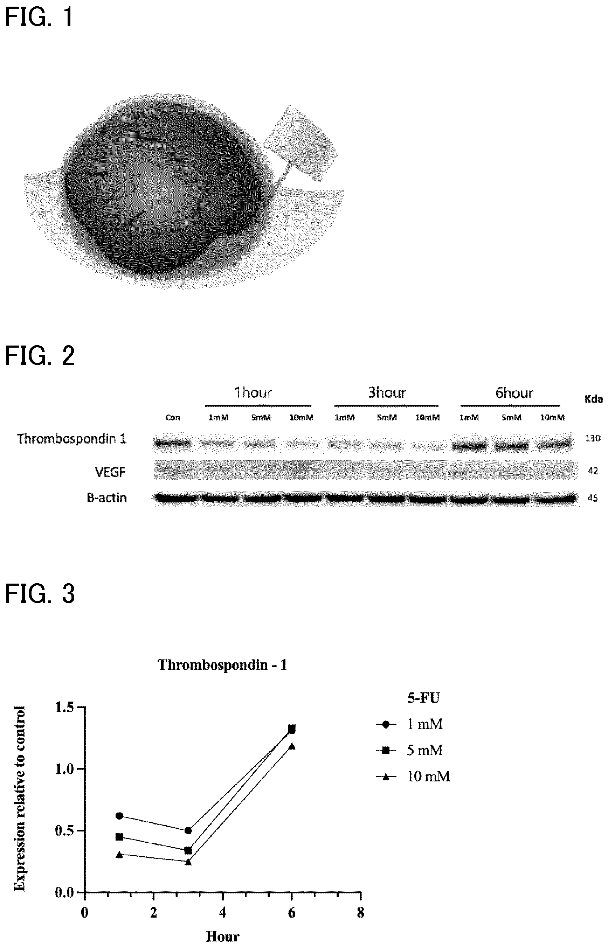

Referring to FIG. 1, for example, the composition may be delivered to the basal region of the tumor via an injector using an endoscope. Specifically, the composition may be injected into the submucosa zone of the normal gastric wall adjacent to the tumor, targeting the basal layer of the tumor. The drug delivered to the vascular endothelial cells of the blood vessels supplying blood to the tumor tissue may induce endothelial cell apoptosis or inhibit neovascularization.

The local injection may deliver a greater amount of the drug not only to the submucosal layer around the tumor but also to the surrounding lymph nodes compared to conventional intravenous administration of anticancer agents. Additionally, local injection may reduce the side effects of anticancer agents compared to systemic administration.

The composition according to an exemplary embodiment may be administered in a pharmaceutically effective amount. The effective dosage level may be determined based on factors such as the type and severity of the patient's disease, the drug's activity, sensitivity to the drug, administration time, route of administration, elimination rate, duration of treatment, co-administered drugs, and other factors well known in the medical field.

A local formulation for solid tumor administration according to an exemplary embodiment may include the composition. Specific examples of solid tumors are the same as described above.

In some embodiments, the composition may be administered as a standalone therapeutic agent or in combination with other therapeutic agents. The components included in a combination anticancer formulation may be administered sequentially or simultaneously and may be administered as a single or multiple doses. Considering all these factors, it is important to administer the minimum effective amount that maximizes therapeutic efficacy while minimizing side effects, which may be readily determined by those skilled in the art.

For example, the dosage of the composition may vary widely depending on factors such as the patient's body weight, age, sex, health status, diet, administration time, administration method, elimination rate, and disease severity. The appropriate dosage may also vary depending on the amount of accumulated drug in the patient's body and/or the specific efficacy of the delivery system used in the present invention. For example, the dosage may range from 0.01 µg to 1 g per kilogram of body weight and may be administered once or multiple times per unit period, such as daily, weekly, monthly, or annually. Alternatively, the composition may be continuously administered over an extended period using an infusion pump. The frequency of repeated administration may be determined based on factors such as drug retention time in the body and drug concentration in the bloodstream. Even after the completion of treatment, the composition may be administered to prevent recurrence depending on the course of disease treatment.

The following examples are provided to describe the present invention in detail.

### <Example>

### Example 1: Verification of the Mechanism of Gastric Cancer Tissue Destruction by 5-FU (Endothelial Cytotoxicity in Tumor Neovascularization)

### 1) Upregulation of Thrombospondin-1 (TSP-1) Expression in Endothelial Cells by 5-FU

Thrombospondin (TSP) is a macromolecule with various functions, and among its isoforms, TSP-1 acts as an angiogenesis inhibitor. The following experiment was conducted to examine changes in TSP-1 expression in response to different concentrations of 5-FU.

Western blot analysis was conducted to confirm the expression of TSP-1 protein in the HUVEC (human umbilical vein endothelial cell) cell line.

HUVECs cultured in petri dishes were lysed using a buffer containing 50 mM Tris (pH 8.0), 150 mM NaCl, 1% Nonidet P-40, 0.5% sodium deoxycholate (SDC), 0.1% sodium dodecyl sulfate (SDS), and 1× protease inhibitor cocktail. The lysates were incubated on ice for 30 minutes, followed by centrifugation at 14,000g for 10 minutes to collect the supernatant.

Protein quantification was performed using a bicinchoninic acid (BCA) protein assay kit (Pierce Chemical, Rockford, IL, USA). A total of 30 µg of protein was subjected to electrophoresis on a 10% SDS-polyacrylamide gel and subsequently transferred onto a nitrocellulose membrane. The membrane was blocked with blocking milk for 1 hour, then incubated with the same TSP-1 antibody used for immunostaining at room temperature for 1 hour. After three washes with Tris buffer containing 0.1% Tween 20 at 15-minute intervals, the membrane was incubated with a secondary antibody specific to the primary antibody at room temperature for 1 hour. The detection was carried out using a chemiluminescent substrate (Amersham Life Science, Arlington Heights, IL, USA). The results are presented in FIG.2.

Protein extracted from HUVECs was used as a positive control. The results of TSP-1 expression in HUVECs according to 5-FU concentration and exposure time are shown in FIG.3. As demonstrated in FIG.3, prolonged exposure to 5-FU and higher drug concentrations led to increased expression levels of TSP-1.

### 2) LDH Release Assay in HUVECs Induced by 5-FU

It has been generally reported that an increase in the expression of thrombospondin-1 (TSP-1) and thrombospondin-2 (TSP-2) induces apoptosis in endothelial cells. To investigate whether the administration of 5-fluorouracil (5-FU) enhances apoptosis in endothelial cells, an LDH release assay was conducted in human umbilical vein endothelial cells (HUVECs). Cytotoxicity was assessed based on the amount of lactate dehydrogenase (LDH) released due to cell membrane damage. The released LDH was quantified using an LDH assay kit (EZ-LDH1000; DoGenBio, Seoul, Korea) according to the manufacturer's protocol.

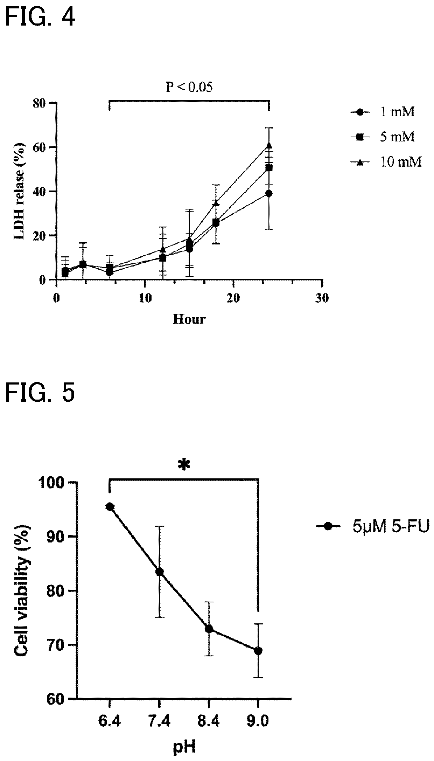

HUVECs (5×10⁴ cells/well) were seeded in a 96-well polystyrene plate and incubated at 37°C for 24 hours. LDH substrate was added to each well, followed by an additional incubation at 37°C for 10 minutes. The plate was centrifuged at 600 g for 5 minutes. A 10-µL aliquot of the supernatant was transferred from the culture plate wells to an analysis plate. Subsequently, 100 µL of LDH reaction mixture reagent was added to the supernatant in each well of the analysis plate. After a 30-minute reaction, absorbance was measured at 450 nm using a microplate reader (Model 680; Bio-Rad). The cytotoxicity of 5-FU on HUVECs was determined based on the amount of LDH released. The results of LDH release in HUVECs according to 5-FU concentration and exposure time are presented in FIG.4.

As shown in FIG.4, the amount of LDH released increased in a time-dependent manner following treatment with 1 mM, 5 mM, and 10 mM 5-FU (P < 0.05).

### 3) Changes in HUVEC Cytotoxicity According to the pH of 5-FU

To evaluate the cytotoxic effect of 5-fluorouracil (5-FU) in human umbilical vein endothelial cells (HUVECs) under different pH conditions, an MTS cell proliferation assay was performed. The cytotoxicity of 5 µM 5-FU at various pH levels in HUVECs was assessed using the CellTiter 96 AQueous One Solution Cell Proliferation Assay (Promega Corp., Madison, WI, USA).

HUVECs were seeded in a 96-well plate at a density of 5×10³ cells/well and incubated for 24 hours. Subsequently, the cells were cultured for an additional 24 hours in Dulbecco's Modified Eagle's Medium (DMEM) containing 5-FU at different pH conditions (pH 6.4, 7.4, 8.4, and 9.0). Following the incubation, the cells were washed twice with phosphate-buffered saline (PBS), after which 100 µL of fresh DMEM growth medium was added, followed by an additional 48-hour incubation. The medium was then replaced with 100 µL of fresh DMEM growth medium, and 20 µL of 3-(4,5-dimethylthiazol-2-yl)-5-(3-carboxymethoxyphenyl)-2-(4-sulfophenyl)-2H-tetrazolium (MTS) solution (Promega Corp., Madison, WI, USA) was added. After an additional 2-hour incubation, the absorbance was measured at 490 nm using a microplate reader (Model 680, BIO-RAD; Spark, TECAN, CA, USA). The results of the MTS cell proliferation assay for 5-FU cytotoxicity in HUVECs according to pH are presented in FIG.5.

As shown in FIG.5, the cytotoxic effect of 5-FU increased as the alkalinity of the drug became stronger, exhibiting significantly greater toxicity under alkaline conditions compared to neutral or acidic 5-FU.

### 4) CD31 and VEGF Staining in Gastric Tumor Tissue Following Local Injection of 5 mg of 5-FU in Nude Mice

To observe changes in vascular tissue within gastric tumor masses after local injection of 5-FU, immunohistochemical staining for CD31 and VEGF was performed.

For the control group, gastric tumor tissue was implanted in the dorsal region of nude mice, and when the tumor reached a size of 1 cm × 1 cm, it was excised and used. For the 5-FU injection group, 5 mg of 5-FU was injected at the base of the tumor when the implanted tumor reached a size of 1 cm × 1 cm. One week after the injection, the gastric tumor mass was excised, and immunohistochemical staining was performed. The immunohistochemical staining method was as follows.

A tissue microarray (TMA) block was prepared from formalin-fixed, paraffin-embedded gastric tumor tissue. The block was sectioned into 3.5 µm-thick slices, which were mounted on slides in a uniform orientation and dried. To enhance immunochemical reactivity, the slides were immersed in 10 mM/L citrate buffer (pH 6.0) and subjected to microwave treatment for 15 minutes. The slides were then washed with triple-distilled water (3'DW) for 5 minutes. To reduce endogenous peroxidase activity present in blood cells within the tissue, the slides were treated at room temperature with 3% H₂O₂ in methanol for 15 minutes. After washing twice with 3'DW for 3 minutes each, a blocking antibody containing normal serum albumin was applied at room temperature for 30 minutes to prevent nonspecific binding. Excess blocking antibody was removed, and the slides were incubated at room temperature for 60 minutes with VEGF antibody (1:100 dilution, sc-7269, Santa Cruz Biotechnology). The slides were washed twice with TBST (Tris-buffered saline with 0.1% Tween^{®} 20 Detergent) for 3 minutes each, followed by incubation at room temperature for 15 minutes with Detection Kit Reagent 1 (HRP Polymer-anti-mouse/rabbit IgG). After another two washes with TBST for 3 minutes each, the slides were incubated at room temperature for 1 minute with Detection Kit Reagent 2 mixture, followed by a 1-2 minute wash with 3'DW (triple-distilled water). For counterstaining, Harris hematoxylin was applied. Smooth muscle endothelial cells were used as a positive control in each staining session.

To evaluate angiogenesis, an antibody specific to CD31 (PECOM-1, sc-376764, Santa Cruz Biotechnology), a marker for vascular endothelial cells, was applied using the same method as for VEGF.

For result analysis, the proportion of the area stained for CD31-positive blood vessels and VEGF-positive cancer cells relative to the total tissue area was calculated, and a comparison was made between the control group and the 5-FU locally injected group. The results are summarized in Table 1.

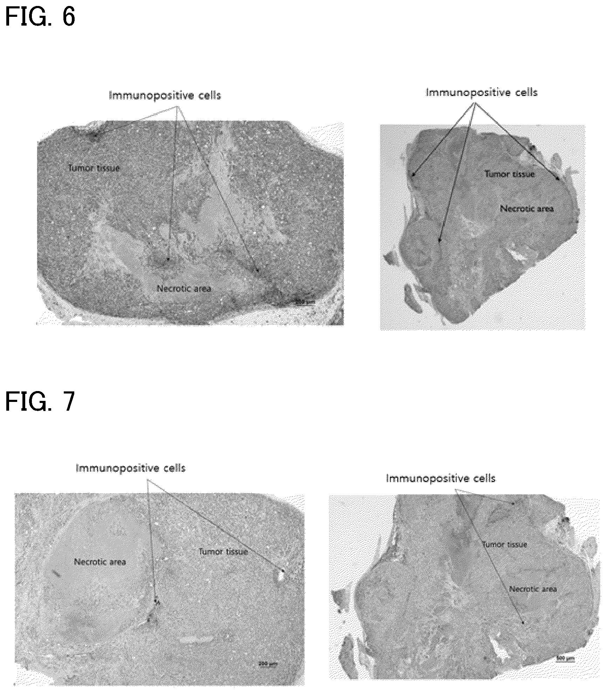

FIG.6 presents the immunostaining results for CD31 in gastric tumor masses one week after injection in the control group (left) and the 5-FU injection group (right). FIG.7 illustrates the VEGF immunostaining results for gastric tumor masses one week after injection in the control group (left) and the 5-FU injection group (right).

**[Table 1]**

| | Total area [*µ*m²] | Immunopositive area [*µ*m²] | Immuno-positive area (%) |
|---|---|---|---|
| Control group (CD31) | 29556494.76 | 257505.634 | 0.87 |
| 5-FU injection group (CD31) | 47304976.18 | 257079.525 | 0.54 |
| Control group (VEGF) | 29556494.76 | 113422.564 | 0.38 |
| 5-FU injection group (VEGF) | 47304976.18 | 46077.917 | 0.10 |

Referring to FIG.6, the immunohistochemical staining results showed that CD31 stained the cytoplasm of vascular endothelial cells brown. In the tumor mass of the 5-FU injection group, compared to the control group, the distribution of necrotic tissue increased, and the stained area ratio of CD31 was lower.

Referring to FIG.7, VEGF was primarily stained as granular deposits localized in the cytoplasm of cancer cells. Compared to the control group, the stained area in the 5-FU injection group appeared smaller. Accordingly, the vascular distribution within the tumor mass of the 5-FU injection group was reduced compared to the control group, and VEGF secretion levels also decreased. These findings suggest that 5-FU injection induced damage to vascular endothelial cells, leading to a reduction in vascular distribution and inhibition of neovascular regeneration, ultimately resulting in tumor necrosis and a decrease in tumor size.

Referring to Table 1, the stained area of CD31 and VEGF, which represent vascular regions, appeared smaller in the 5-FU injection group compared to the control group.

### 5) In Vivo Experiment to Evaluate the Response of Anticancer Drugs After Xenografting Human Gastric Cancer Cells on the Back of Nude Mice

The animals used in the experiment were six male nude mice (Crj: BALB/c-nu/nu mice, male) aged five weeks and weighing approximately 30 grams, purchased from Orient. The mice were subjected to a one-week acclimation period in the laboratory before use. Each nude mouse was subcutaneously xenografted with 5 × 10⁶ human gastric cancer cells suspended in 100 µL of PBS on the dorsal subcutaneous layer. The tumor size was periodically measured, and when it reached a diameter of 1 cm, the drug administration experiment was conducted.

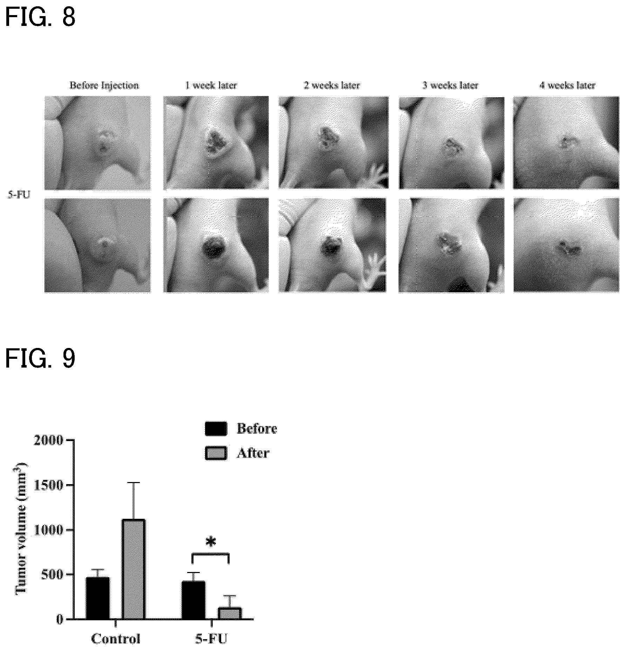

The experimental animals were divided into two groups. One group served as the control group, receiving only physiological saline, while the other group was administered 5 mg of 5-FU at pH 8.4. The drug was injected 0.2 cc adjacent to the tumor three times at one-week intervals. After one month, the major and minor axes of the tumors growing on the dorsal epidermis were measured. The tumor volume before and after drug administration was calculated using the formula mean tumor volume = (major axis × minor axis²) / 2 (mm³) and analyzed for comparison. The results are presented in FIG.8 and FIG.9.

In FIG.8, the tumor size decreased four weeks after drug administration compared to before injection (P < 0.05).

In FIG.9, the tumor size was reduced in the group administered alkaline 5-FU compared to the control group that received PBS (P < 0.05).

### 6) Observation of Pathological Changes in the Subcutaneous Muscle Layer After Subcutaneous Injection of 5-FU

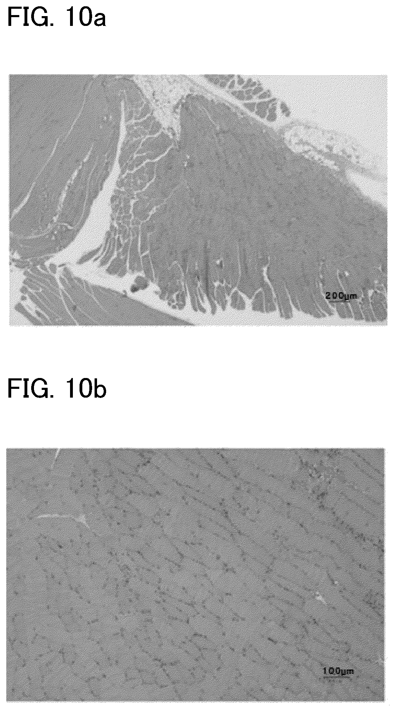

To observe pathological changes in the surrounding tissue after injecting 5 mg of pH 8.4 5-FU near the tumor, the drug was locally administered to nude mice. After 24 hours, tissues, including muscle layers, were collected and subjected to H&E staining to examine degeneration, necrosis, inflammation, and fibrosis in the muscle layer. The extent of each pathological change was scored as follows: 0 = absent, 1 = mild, 2 = moderate, 3 = severe, and the results are shown in FIG.10a and FIG.10b. FIG.10a presents observations at 40× magnification, while FIG.10b presents observations at 100× magnification.

As shown in FIG.10a and FIG. 10b, no pathological changes were observed in the subcutaneous muscle layer at the injection site of 5-FU (score = 0).

### Example 2: Verification of the Mechanism of Gastric Tumor Tissue Necrosis Promotion by L-NMMA

### 1) Changes in HUVEC Cytotoxicity According to L-NMMA Concentration

To investigate the apoptotic effect of 5-FU and L-NMMA concentrations on HUVECs, an MTS cell proliferation assay was conducted.

Cytotoxicity of 5 µM 5-FU under various concentrations of L-NMMA in HUVECs was measured using the CellTiter 96 AQueous One Solution Cell Proliferation Assay. HUVECs were seeded at a density of 5 × 10³ cells/well in a 96-well plate and incubated for 24 hours. Subsequently, the cells were incubated in DMEM medium containing various concentrations of L-NMMA for 24 hours. The cells were then washed twice with PBS, followed by the addition of 100 µL of fresh growth DMEM medium, and incubated for an additional 48 hours. The medium was replaced with 100 µL of fresh growth DMEM medium, after which 20 µL of 3-(4,5-dimethylthiazol-2-yl)-5-(3-carboxymethoxyphenyl)-2-(4-sulfophenyl)-2H-tetrazolium (MTS) was added. After an additional 2-hour incubation, absorbance at 490 nm was measured using a BIO-RAD Model 680 microplate reader (Spark, TECAN, CA, USA).

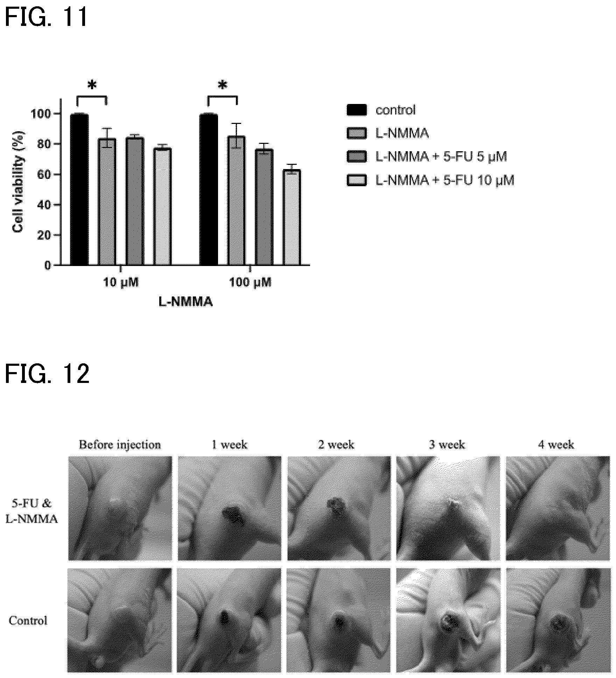

The results are shown in FIG. 11. Referring to FIG.11, a reduction in cell viability was observed upon administration of L-NMMA alone, and an additional apoptotic effect was confirmed when co-administered with 5-FU.

### 2) In vivo experiment to observe the response of an anticancer drug after xenotransplantation of human gastric cancer cells into the back of nude mice

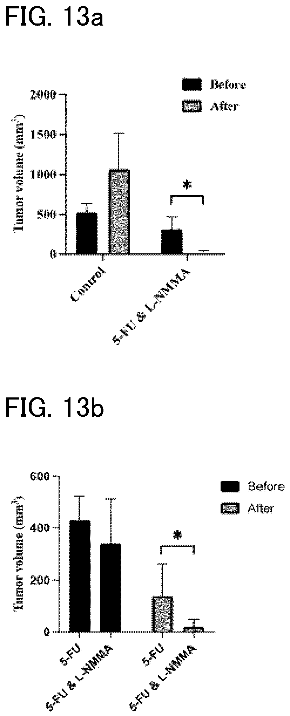

The animals used in the experiment were two male nude mice (Crj: BALB/c-nu/nu mice, male), approximately 5 weeks old and weighing an average of 30 g, produced by Orient. The mice underwent an acclimation period of one week at the research facility before use. Each nude mouse was subcutaneously implanted with 5 × 10⁶ human gastric cancer cells/100 µL (PBS) in the dorsal region. Tumor size was measured periodically, and when the tumor reached a diameter of 1 cm, drug administration experiments were conducted. The experimental animals were divided into two groups. One group served as the control group and received only normal saline, while the other group received 5 mg of 5-FU at pH 8.4 and 0.1 mg of L-NMMA. These drugs were injected at 0.2 cc adjacent to the tumor, administered three times at one-week intervals. After one month, the length and width of the tumors growing on the dorsal skin were measured, and the tumor volume before and after drug administration was calculated using the formula: mean tumor volume = (length × width²) / 2 (mm³) for comparative analysis. The results are shown in FIG.12, FIG.13a, and FIG.13b.

Referring to FIG.12, FIG.13a, and FIG.13b, the tumor size significantly decreased after the administration of 5-FU and L-NMMA. The reduction in tumor size was greater compared to the administration of 5-FU alone.

### Example 3: Verification of the Tumoricidal Effect of Bevacizumab on Gastric Cancer Tissue

### 1) In vivo experiment to observe the response of the anticancer drug (bevacizumab) after xenotransplantation of human gastric cancer cells into the back of nude mice.

The animals used in the experiment were three male nude mice (Crj: BALB/c-nu/nu, male), each weighing approximately 30 g and five weeks old, produced by Orient. The mice underwent a one-week acclimatization period in the laboratory before use. Human gastric cancer cells (5 × 10⁶ cells/100 µL in PBS) were xenografted into the subcutaneous fat layer on the dorsal side of each nude mouse. The tumor size was measured periodically, and drug administration was initiated when the tumor reached a diameter of 1 cm.

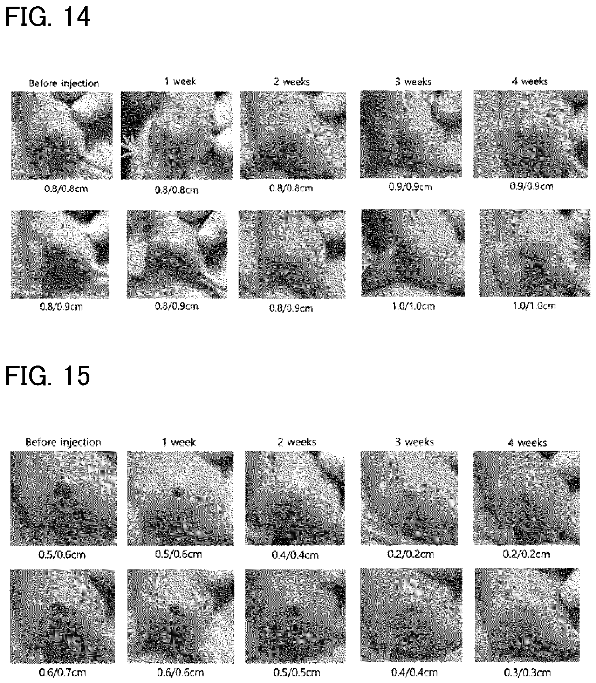

The experimental animals were administered 1 mg of bevacizumab by injecting 0.2 cc adjacent to the tumor three times at one-week intervals. After one month, the length and width of the tumors growing on the dorsal skin were measured. The tumor volume before and after drug administration was calculated using the formula: mean tumor volume = (length × width²) / 2 (mm³) for comparative analysis. The results are shown in FIG.14.

As shown in FIG.14, the tumor size remained nearly unchanged four weeks after drug administration compared to before injection (P > 0.05). The tumor size did not change for the following two months and began to increase in the third month.

### 2) In Vivo Experiment to Evaluate the Response of Anticancer Drugs(Alkaline 5-FU and Bevacizumab) After Xenografting Human Gastric Cancer Cells on the Back of Nude Mice

The experimental animals used in this study were three male nude mice (Crj: BALB/c-nu/nu mice, male) aged five weeks, each weighing approximately 30 g, obtained from Orient Bio. The animals were acclimated for one week in the research facility before use. Human gastric cancer cells (5×10⁶ cells/100 µL in PBS) were implanted into the subcutaneous fat layer of the dorsal region of each nude mouse. Tumor size was periodically measured, and drug administration experiments were conducted when the tumor diameter reached 1 cm.

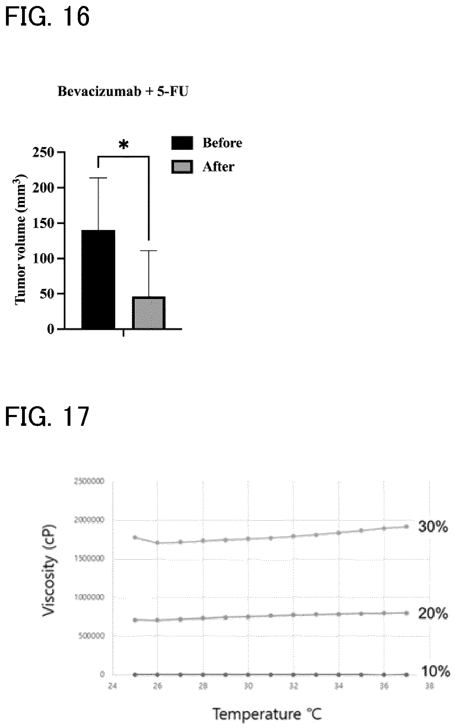

The experimental animals were administered 0.2 mg of bevacizumab and 5 mg of pH 8.4 5-FU by injecting 0.2 cc adjacent to the tumor three times at one-week intervals. After one month, the length and width of the tumors growing on the dorsal skin were measured. The tumor volume before and after drug administration was calculated using the formula: mean tumor volume = (length × width²) / 2 (mm³) for comparative analysis. The results are shown in FIG.15 and FIG.16.

Referring to FIG.15 and FIG.16, the tumor size decreased four weeks after drug administration compared to before injection (P<0.05). A distinct pattern of tumor regression was observed. The overall tumor size remained stable initially, followed by necrosis starting from the center and progressively spreading outward.

### Example 4: Treatment of Gastric Cancer Using Local Injection of Sustained-Release 5-FU Sol-Gel

### 1) Determination of the Concentration of Pluronic F-127 (P407) for the Preparation of 5-FU-Sol-Gel

To evaluate the viscosity of P407 based on temperature and concentration, the viscosity was measured using a Brookfield viscometer while increasing the temperature from approximately 24°C to 38°C. The concentrations of P407 were set at 10%, 20%, and 30%. The results are shown in FIG.17.

Referring to FIG.17, a P407 concentration of 30% was confirmed to be necessary for gelation to occur at 37°C. Additionally, at concentrations exceeding 30%, the viscosity became excessively high, making local injection of the drug difficult. Therefore, 30% was determined to be the most appropriate concentration.

### 2) Drug Release Test of 5-FU-Sol-Gel (UV-VIS Spectrophotometer)

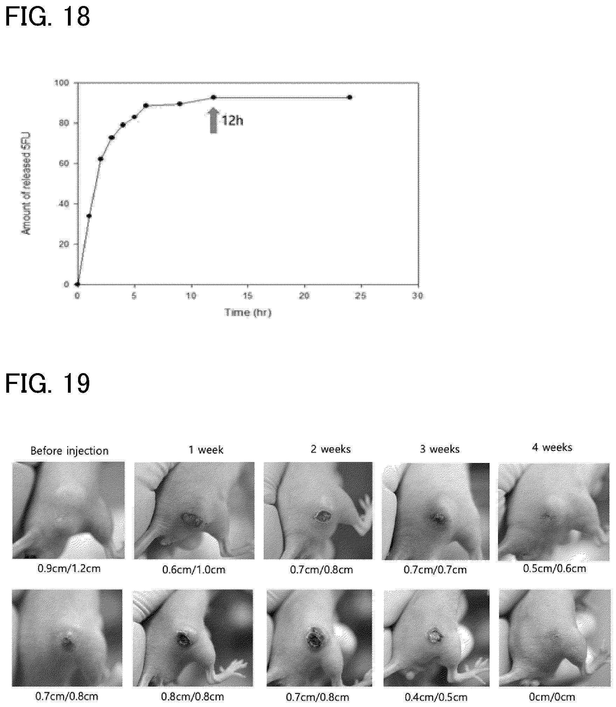

A drug release experiment was conducted using 5 mL of PBS and 0.76 mL of gel (2 mm thickness, 30% P407, 4.3 mg 5-FU). Changes were observed at 1, 2, 3, 4, 5, 6, 9, 12, and 24 hours under a 37°C environment. The results are shown in FIG.18.

Referring to FIG.18, complete drug release was confirmed after 12 hours.

### 3) In Vivo Experiment to Evaluate the Response of Anticancer Drugs After Xenografting Human Gastric Cancer Cells on the Back of Nude Mice

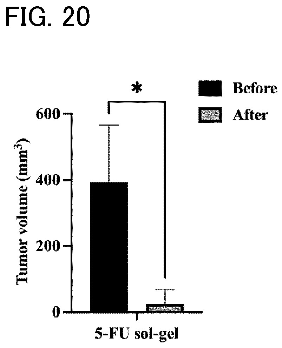

The experimental animals used were five male nude mice (Crj: BALB/c-nu/nu, male), each approximately 5 weeks old and weighing around 30 g, obtained from Orient. The animals were acclimated in the laboratory for one week before use. Each nude mouse was subcutaneously implanted with 5 × 10⁶ human gastric cancer cells/100 µL (PBS) in the dorsal subcutaneous fat layer. The tumor size was periodically measured, and when it reached a diameter of 1 cm, drug administration experiments were performed. A 0.2 cc solution (5 mg 5-FU) containing 30% P407 and 2.5% pH 8.4 5-FU was injected adjacent to the tumor three times at one-week intervals. After one month, the length and width of the tumors growing on the dorsal skin were measured. Tumor volume before and after drug administration was calculated using the formula: mean tumor volume = (length × width²) / 2 (mm³) for comparative analysis. The results are shown in FIG.19 and FIG.20.

Referring to FIG.19 and FIG.20, administration of the drug to the lower part of the gastric tumor resulted in near-complete necrosis of the malignant tumor. Furthermore, a comparison of tumor volume before and after drug administration confirmed a significant reduction in tumor size (P < 0.05).

## Claims

1. A pharmaceutical composition for tumor vascular disruption, comprising alkaline 5-fluorouracil and a nitric oxide synthase inhibitor.

2. The pharmaceutical composition for tumor vascular disruption according to Claim 1, wherein the nitric oxide synthase inhibitor comprises one or more selected from L-NMMA (NG-Monomethyl-L-arginine), L-NAME (NG-Nitro-L-arginine methyl ester), L-NA (Nitro Arginine), and 7NI (Nitroindazole).

3. The pharmaceutical composition for tumor vascular disruption according to Claim 1, wherein the nitric oxide synthase inhibitor comprises L-NMMA (NG-Monomethyl-L-arginine).

4. The pharmaceutical composition for tumor vascular disruption according to Claim 1, wherein the alkalinity is in the range of pH 8 to 9.

5. The pharmaceutical composition for tumor vascular disruption according to Claim 1, wherein the alkaline 5-fluorouracil is in a form in which 5-fluorouracil is dissolved in an alkaline solvent.

6. The pharmaceutical composition for tumor vascular disruption according to Claim 1, wherein the concentration of the alkaline 5-fluorouracil is in the range of 0.1 to 600 mM in the total composition.

7. The pharmaceutical composition for tumor vascular disruption according to Claim 1, wherein the alkaline 5-fluorouracil increases thrombospondin-1 protein expression in tumor vascular endothelial cells, thereby inducing apoptosis of the endothelial cells.

8. The pharmaceutical composition for tumor vascular disruption according to Claim 1, wherein the concentration of the nitric oxide synthase inhibitor is in the range of 0.1 to 300 mM in the total composition.

9. The pharmaceutical composition for tumor vascular disruption according to Claim 1, wherein the nitric oxide synthase inhibitor constricts tumor blood vessels and promotes thrombosis within the tumor vasculature damaged by 5-fluorouracil, thereby occluding the blood vessels.

10. The pharmaceutical composition for tumor vascular disruption according to Claim 1, further comprising one or more selected from bevacizumab, capric acid or a pharmaceutically acceptable salt thereof, and poloxamer.

11. The pharmaceutical composition for tumor vascular disruption according to Claim 1, further comprising one or more selected from cytotoxic anticancer agents, targeted anticancer agents, and immuno-oncology agents.

12. The pharmaceutical composition for tumor vascular disruption according to Claim 1, wherein the tumor comprises a solid tumor.

13. The pharmaceutical composition for tumor vascular disruption according to Claim 1, wherein the composition is administered locally by injection around the tumor.

14. The pharmaceutical composition for tumor vascular disruption according to Claim 13, wherein the area around the tumor is a submucosal layer where tumor blood vessels are located.

15. A local formulation for solid tumor administration, comprising the pharmaceutical composition for tumor vascular disruption according to Claim 1.
